# EUROPEAN PATENT SPECIFICATION

(11) **EP 1 002 552 B1**
(45) Date of publication and mention of the grant of the patent: **27.06.2007**
(21) Application number: 99309138.8
(22) Date of filing: 17.11.1999
(51) Int. Cl.: A61M 16/16

(54) **Medical respiratory apparatus**
Medizinisches Beatmungsgerät
Appareil respiratoire

(30) Priority: 18.11.1998 GB 9825146
(43) Date of publication of application: 24.05.2000
(60) Divisional application: 07103642.0
(73) Proprietor: Intersurgical AG, Vaduz (LI)
(72) Inventor: Booth, Christopher Edgerley, Ashton, Cheshire CH3 8AB (GB); Jassell, Surinderjit Kumar, Northolt, Middlesex UB8 6DE (GB)
(74) Representative: Jones, Stephen Anthony

(56) References cited:
- EP-A- 0 587 380
- US-A- 3 817 246
- US-A- 4 121 686
- US-A- 4 243 396

## Description

This invention relates to improvements in medical respiratory apparatus of the kind used in patient ventilation, and more particularly to improvements in a combined nebuliser and humidifier.

Patients in hospitals must frequently be provided with an oxygen-enriched air supply, and to avoid drying internal tissue moisture is preferably added to the mixture by means of a nebuliser, which atomises water in a gas jet before supplying the mixture to a face mask. A problem with existing equipment for this purpose is noise emission. This may not be loud but it is irritating to a patient attempting to sleep, for example in an otherwise silent environment, and when numerous nebulisers/humidifiers are in use in the same hospital ward the aggregate noise emission reaches unacceptable levels.

A principal object of the present invention is to address this problem in a simple and economically viable manner.

EP 0587380 discloses a nebuliser including a baffle for reducing noise emission from an atmospheric air inlet of the nebuliser.

In accordance with one aspect of the present invention there is provided respiratory apparatus for medical use comprising a housing of a first material having a first inlet for connection to a source of gas under pressure, second inlet means for the entrainment of air and an outlet for supplying the gaseous mixture to a patient, the interior of the housing being at least partially lined by a tubular baffle, characterised in that the tubular baffle is of a second material having a greater density than the first material of the housing, and the tubular baffle provides a funnel-shaped pathway for the mixture between the inlets and the outlet.

Both the housing and the baffle are most conveniently formed in plastics materials, eg using suitable moulding techniques such as injection moulding etc. The density of the material used for the baffle may be increased by incorporating into that material a particulate filler of higher density than the plastics material. A variety of such fillers may be suitable, including such materials as powdered metals, and inorganic fillers such as fly ash. A particularly preferred filler is particulate iron ore.

Preferably, the material of the baffle has a density at least 30% greater than that of the housing material, more preferably at least 50% greater, say 50% to 150% greater. Typically, the material of the baffle has a density of from about 1.4 to 2.4g/cm³. The material used in the housing may have a density of from about 0.95 to 1.13g/cm³.

The baffle is preferably moulded from a plastics compound of which a greater proportion is an iron ore filler. The compound may have a base of polypropylene and the compound is preferably approximately 60% iron ore to 40% polypropylene. The thickness of the baffle material is preferably in the range 2 to 3mm.

The first inlet may be located in the top of the housing, the outlet may extend radially of the housing between the top and bottom thereof and the second inlet may be disposed radially of the housing between the top thereof and the outlet, the baffle having a generally cylindrical component to line the interior of the housing below the top thereof and a generally trumpet-shaped component coaxial with the cylindrical component and joined thereto at the wider periphery of the trumpet-shaped portion, the narrower-diameter portion of the trumpet-shaped component extending away from the top of the housing beyond the cylindrical component.

A bottom portion of the housing may be a water reservoir from which a tube extends upward through the baffle to a nozzle aligned with gas incoming through the first inlet, thereby to add atomised water to the mixture going to the outlet.

The narrower-diameter portion of the trumpet-shaped component of the baffle may have a radial opening which confronts the interior of the housing at a position substantially diametrically opposite to the outlet.

The junction between the cylindrical and trumpet-shaped components of the baffle may be between top and bottom ends of the cylindrical component and the latter may be cut away where aligned both with the second inlet and with the outlet.

The portion of the cylindrical component which extends below the junction with the trumpet-shaped component may have longitudinal slits in circumferentially spaced relation.

The narrower-diameter portion of the trumpet-shaped component may terminate in a coaxial opening dimensioned closely to surround the water tube.

Further problem associated with existing equipment is that the ring which is turned to adjust the proportion of air admitted to the nebuliser is freely rotatable. It may be accidentally knocked, thereby altering the intended adjustment and this may pass unnoticed.

Further object of the present invention is to address this problem also.

Therefore in accordance with further aspect of the present invention there is provided respiratory apparatus for medical use comprising a housing having a first inlet for connection to a source of gas under pressure, second inlet means for the entrainment of air and an outlet for supplying the gaseous mixture to a patient, wherein the second inlet means is adjustable by means of a ring rotatable around the upper part of the housing, the ring being spring loaded to bring a lower end thereof into contact with a shoulder of the housing, said lower end and said shoulder having formations which will inter-engage to prevent rotation of the ring but which may be disengaged to permit rotation of the ring if the ring is lifted against the action of the spring.

The ring may be a double-walled structure, closed at one end whereby air is admitted to the second inlet means from an open end of the ring between the walls of the ring, the inner wall of the ring having an opening which can be brought into and out of alignment with the second inlet means of the housing as the ring is rotated.

The second inlet means may comprise a plurality of apertures spaced circumferentially around the housing at the same horizontal level below the top of the housing, and the inner wall of the ring may have a plurality of apertures the circumferential spacing of which corresponds with the circumferential spacing of said plurality of apertures.

A preferred embodiment of the present invention will now be described by way of non-limitative example with reference to the accompanying Drawings, in which:

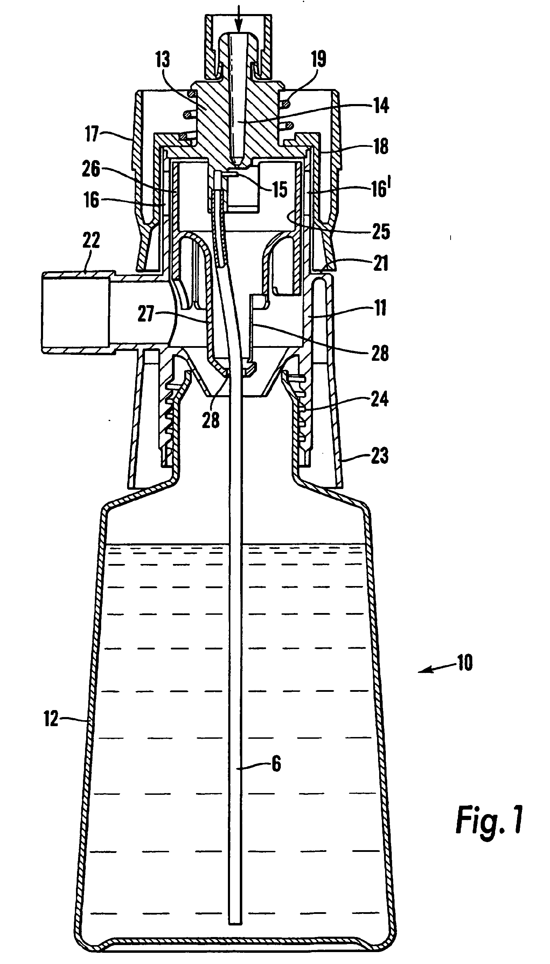

- Figure 1: is a sectional elevation of a nebuliser/humidifier in accordance with the invention;
- Figure 2: is a perspective view of an upper part of the apparatus of Figure 1, partly broken away for purposes of illustration, and
- Figure 3: is a perspective view of the baffle incorporated in the apparatus of Figures 1 and 2.

The nebuliser/humidifier 10 illustrated comprises a housing 11 mounted on a water reservoir 12. At the top of the housing 11 is a first inlet 13 which has standard connections for connecting the interior of the housing to a source of gas under pressure, such as oxygen. As is known *per se* this enters the housing through a nozzle 14 which is at right angles to a nozzle 15 at the upper end of a tube 6 which depends into the water in reservoir 12, so that gas under pressure emerging from nozzle 14 atomises water emerging from nozzle 15 to add water vapour in the form of a fine mist to the gases in the housing.

Below its top the housing 11 has two radial openings or windows 16 and 16' at diametrically opposite positions. These are controlled by rotation of a ring 17 surrounding the upper part of the housing to vary the proportion of air admitted to the interior of housing 11 to mix with gas from the nozzle 14 and water vapour from the nozzle 15.

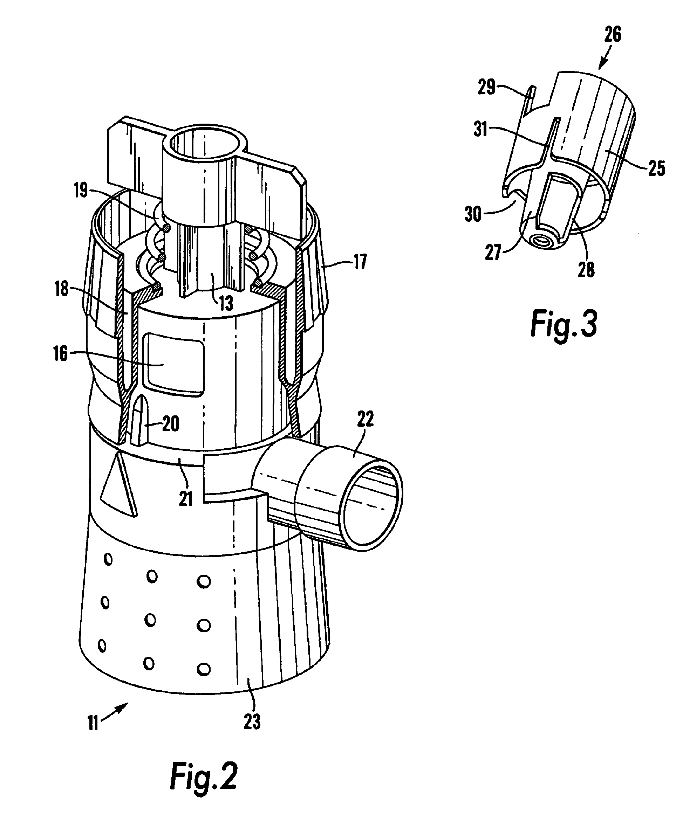

In accordance with one feature of the present invention the ring 17 is a double-walled structure closed at the bottom end of the ring and open at its upper end to admit air between the walls. The inner wall 18 has openings or windows not visible in the Drawings which correspond in position and size to the openings 16,16'. The ring 17 can be rotated between a position in which its windows are fully aligned with the windows 16,16' to admit a maximum volume of air to the interior of the housing 11 and a position in which the windows 16,16' are obturated by the ring 17. A spring 19 biasses the ring 17 downward so that lugs, one of which is shown at 20 in Figure 2, which are distributed circumferentially around a shoulder 21 of the housing, may enter recesses (not shown) in the bottom of the ring 17. The engagement of the lugs 20 in these recesses normally prevents rotation of the ring 17 relative to the housing 11, but when it is desired to vary the volume of air admitted to the interior of the housing the ring 17 can be lifted against the action of the spring 19, turned to a different angular position and then released so that the lugs and recesses re-engage with the ring in the new position.

At a radial position below the openings 16,16' the housing 11 has an outlet 22 which is adapted to receive one end of an air line (not shown) the other end of which terminates in equipment such as a face mask (not shown) whereby the mixture of gases and vapour leaving the housing 11 may be administered to a patient.

On a level with and below the outlet 22 the housing 11 has a perforated skirt 23 which in use conceals the screw-threaded connection 24 between the top of the reservoir 12 and the bottom of the housing 11. The housing 11 may be unscrewed from the reservoir 12 to replenish the water in the reservoir.

In accordance with the present invention the interior of the housing 11 is lined by a cylindrical component 25 of a baffle 26 which also has a trumpet-shaped component 27 extending below the cylindrical component coaxially within the housing 11. The baffle 26 is of a material having better sound-deadening characteristics than the material from which the housing 11 is made. Specifically the material of the baffle 26 is of greater density than that from which the housing 11 is made. A suitable material for the baffle 26 is a compound of polypropylene and an iron ore filler such as that known as "Magnetite" in proportions of around 60% of the latter. The material of the baffle may have a thickness in the range 2 to 3mm.

The larger-diameter periphery of the trumpet 27 is integral with the cylindrical component 25 at a position about one third the length of the cylindrical component measured from its bottom end. Thus the narrower-diameter part of the trumpet 27 extends downwardly from the cylindrical component 25 coaxially within the housing 11 and terminates at its bottom end in an opening 28 which is just large enough to allow passage through it of the tube 6. The narrower-diameter part of the trumpet also has a much larger radial opening 28 which opens toward the internal wall of the housing 11 diametrically opposite to the outlet 22. Through this opening 28 of the baffle 26 the mixture of gas, air and water vapour is impacted against the interior wall of the housing 11 and then passes around the baffle 26 to exit through the outlet 22.

Notches 29 and 30 in the cylindrical component 25 of the baffle 26 are aligned respectively with the windows 16,16' and with the outlet 22. The lower part of the cylindrical component 25 below its junction with the trumpet-shaped component 27 is formed with four longitudinal slits in circumferentially spaced relation, one of which is visible at 31 in Figure 3.

## Claims

1. Respiratory apparatus for medical use comprising a housing (11) of a first material having a first inlet (13) for connection to a source of gas under pressure, second inlet means for the entrainment of air and an outlet (22) for supplying the gaseous mixture to a patient, the interior of the housing (11) being at least partially lined by a tubular baffle (26),
**characterised in that** the tubular baffle (26) is of a second material having a greater density than the first material of the housing (11), and the tubular baffle (26) provides a funnel-shaped pathway for the mixture between the inlets (13,16,16') and the outlet (22).

2. Apparatus as claimed in Claim 1, wherein both the housing (11) and the baffle (26) are formed in plastics material.

3. Apparatus as claimed in Claim 1 or Claim 2, wherein the baffle (26) is formed in a plastics material containing a particulate filler of higher density than the plastics material.

4. Apparatus as claimed in claim 3, wherein the baffle (26) is moulded from a plastics compound of which a greater proportion is an iron ore filler.

5. Apparatus as claimed in claim 4, wherein said compound has a base of polypropylene and the compound is approximately 60% iron ore to 40% polypropylene.

6. Apparatus as claimed in any preceding claim, wherein the thickness of the baffle material is in the range 2 to 3mm.

7. Apparatus as claimed in any preceding claim, wherein the material of the baffle (26) has a density of 1.4 to 2.4g/cm³, and the material of the housing (11) has a density of 0.95 to 1.13g/cm³.

8. Apparatus as claimed in any preceding claim, wherein the baffle (26) is of a material with a density at least 30% greater than that of the housing (11).

9. Apparatus as claimed in any preceding claim, wherein the first inlet (13) is located in the top of the housing (11), the outlet (22) extends radially of the housing (11) between the top and bottom thereof and the second inlet (16,16') being disposed radially of the housing (11) between the top thereof and the outlet (22), the baffle (26) having a generally cylindrical component (25) to line the interior of the housing (11) below the top thereof and a generally trumpet-shaped component (27) coaxial with the cylindrical component (25) and joined thereto at the wider periphery of the trumpet-shaped portion (27), the narrower-diameter portion of the trumpet-shaped component (27) extending away from the top of the housing (11) beyond the cylindrical component (25).

10. Apparatus as claimed in Claim 9, wherein a bottom portion of the housing (11) is a water reservoir (12) from which a tube (6) extends upward through the baffle (26) to a nozzle (15) aligned with gas incoming through the first inlet (13), thereby to add atomised water to the mixture going to the outlet (22).

11. Apparatus as claimed in Claim 9 or Claim 10, wherein the narrower-diameter portion of the trumpet-shaped component (27) of the baffle (26) has a radial opening (28) which confronts the interior of the housing (11) at a position substantially diametrically opposite to the outlet (22).

12. Apparatus as claimed in any one of Claims 9 to 11, wherein the junction between the cylindrical (25) and trumpet-shaped components (27) of the baffle (26) is between top and bottom ends of the cylindrical component (25) and the latter is cut away where aligned both with the second inlet (16,16') and with the outlet (22).

13. Apparatus as claimed in any one of Claims 9 to 12, wherein the portion of the cylindrical component (25) which extends below the junction with the trumpet-shaped component (27) has longitudinal slits (31) in circumferentially spaced relation.

14. Apparatus as claimed in any one of Claims 9 to 13, wherein the narrower-diameter portion of the trumpet-shaped component (27) terminates in a coaxial opening (28) dimensioned closely to surround the water tube (6).

15. Apparatus as claimed in any preceding claim, wherein the second inlet means (16,16') is adjustable by means of a ring (17) rotatable around the upper part of the housing (11), the ring (17) being spring loaded to bring a lower end thereof into contact with a shoulder (21) of the housing (11), said lower end and said shoulder (21) having formations (20) which will inter-engage to prevent rotation of the ring (17) but which may be disengaged to permit rotation of the ring (17) if the ring (17) is lifted against the action of the spring (19).

16. Apparatus as claimed in Claim 15, wherein the ring (17) is a double-walled structure, closed at one end whereby air is admitted to the second inlet means (16,16') from an open end of the ring (17) between the walls of the ring (17), the inner wall of the ring (17) having an opening which can be brought into and out of alignment with the second inlet means (16,16') of the housing (11) as the ring (17) is rotated.

17. Apparatus as claimed in Claim 15 or Claim 16, wherein the second inlet means comprises a plurality of apertures (16,16') spaced circumferentially around the housing (11) at the same horizontal level below the top of the housing (11), and the inner wall of the ring (17) has a plurality of apertures the circumferential spacing of which corresponds with the circumferential spacing of said plurality of apertures (16,16').

## Patentansprüche

1. Medizinisches Beatmungsgerät umfassend ein Gehäuse (11) aus einem ersten Werkstoff, das einen ersten Einlass (13) zur Verbindung mit einer Druckgasquelle aufweist, zweite Einlassmittel zum Zumischen von Luft, und einen Auslass (22) zum Zuführen des Gasgemisches zu einem Patienten, wobei das Innere des Gehäuses (11) wenigstens teilweise von einem rohrförmigen Schalldämpfer (26) umkleidet ist,
**dadurch gekennzeichnet,**
**dass** der rohrförmige Schalldämpfer (26) aus einem zweiten Werkstoff besteht, der eine höhere Dichte als der erste Werkstoff des Gehäuses (11) aufweist, und dass der rohrförmige Schalldämpfer (26) einen trichterförmigen Pfad für das Gemisch zwischen den Einlässen (13, 16, 16') und dem Auslass (22) bereitstellt.

2. Gerät gemäß Anspruch 1, wobei das Gehäuse (11) und der Schalldämpfer (26) aus Kunststoff hergestellt sind.

3. Gerät gemäß Anspruch 1 oder 2, wobei der Schalldämpfer (26) aus einem Kunststoff hergestellt ist, der einen Füller in Form von Partikeln mit einer höheren Dichte als der Kunststoff enthält.

4. Gerät gemäß Anspruch 3, wobei der Schalldämpfer (26) aus einer Kompoundmasse geformt ist, deren größerer Anteil ein Eisenerzfüller ist.

5. Gerät gemäß Anspruch 4, wobei die Kompoundmasse eine Basis aus Polypropylen aufweist und die Kompoundmasse ungefähr zu 60% Eisenerz und 40% Polypropylen enthält.

6. Gerät gemäß einem der vorangehenden Ansprüche, wobei die Dicke des Schalldämpfermaterials in einem Bereich von 2 bis 3 mm liegt.

7. Gerät gemäß einem der vorangehenden Ansprüche, wobei der Werkstoff des Schalldämpfers (26) eine Dichte von 1,4 bis 2,4 g/cm³ und der Werkstoff des Gehäuses (11) eine Dichte von 0,95 bis 1,13 g/cm³ aufweist.

8. Gerät gemäß einem der vorangehenden Ansprüche, wobei der Schalldämpfer (26) aus einem Werkstoff mit einer Dichte besteht, die wenigstens 30% höher als die des Werkstoffs ist, aus dem das Gehäuse (11) besteht.

9. Gerät gemäß einem der vorangehenden Ansprüche, wobei der erste Einlass (13) an der Oberseite des Gehäuses (11) angeordnet ist und der Auslass (22) sich radial bezüglich des Gehäuses (11) zwischen dessen Oberseite und Unterseite erstreckt und wobei der zweite Einlass (16, 16') radial bezüglich des Gehäuses (11) zwischen dessen Oberseite und dem Auslass (22) angeordnet ist und wobei der Schalldämpfer (26) eine im allgemeinen zylindrische Komponente (25), die das Innere des Gehäuses (11) unter dessen Oberseite umkleidet, und eine im allgemeinen trompetenförmige Komponente (27) koaxial zu der zylindrischen Komponente (25) aufweist und mit dieser an dem größeren Umfang des trompetenförmigen Abschnitts (27) verbunden ist, wobei der Abschnitt der trompetenförmigen Komponente (27) mit kleinerem Durchmesser sich von der Oberseite des Gehäuses (11) über die zylindrische Komponente (25) weg erstreckt.

10. Gerät gemäß Anspruch 9, wobei ein Bodenabschnitt des Gehäuses (11) ein Wasserbehälter (12) ist, von dem sich ein Rohr (6) nach oben durch den Schalldämpfer (26) zu einer Düse (15) erstreckt, die zu dem Gas, das durch den ersten Einlass (13) eintritt, ausgerichtet ist, wodurch zerstäubtes Wasser zu dem Gemisch, das zu dem Auslass (22) geleitet wird, hinzugegeben wird.

11. Gerät gemäß Anspruch 9 oder 10, wobei der Abschnitt kleineren Durchmessers der trompetenförmigen Komponente (27) des Schalldämpfers (26) eine radiale Öffnung (28) aufweist, die der Innenseite des Gehäuses (11) an einer zu dem Auslass (22) im wesentlichen diametralen Position angeordnet ist.

12. Gerät gemäß einem der Ansprüche 9 bis 11, wobei die Verbindung zwischen der zylindrischen Komponente (25) und der trompetenförmigen Komponente (27) des Schalldämpfers (26) zwischen dem oberen Ende und dem unteren Ende der zylindrischen Komponente (25) angeordnet ist, und wobei das untere Ende der zylindrischen Komponente (25) an der Stelle abgeschnitten ist, die sowohl zu dem zweiten Einlass (16, 16') als auch dem Auslass (22) ausgerichtet ist.

13. Gerät gemäß einem der Ansprüche 9 bis 12, wobei der Abschnitt der zylindrischen Komponente (25), der sich von der Verbindung mit der trompetenförmigen Komponente (27) nach unten erstreckt, Längsschlitze (31) aufweist, die umfangsmäßig beabstandet sind,.

14. Gerät gemäß einem der Ansprüche 9 bis 13, wobei der Abschnitt der trompetenförmigen Komponente (27) kleineren Durchmessers in einer koaxialen Öffnung (28) endet, die so eng dimensioniert ist, dass sie das Wasserrohr (6) umschließt.

15. Gerät gemäß einem der vorangehenden Ansprüche, wobei die zweiten Einlassmittel (16, 16') mittels eines Rings (17) einstellbar sind, der um einen oberen Abschnitt des Gehäuses (11) drehbar ist, und wobei der Ring (17) so federbelastet ist, dass sein unteres Ende mit einer Schulter (21) des Gehäuses (11) in Kontakt bringbar ist, wobei das untere Ende und die Schulter (21) Ausformungen (20) aufweisen, die miteinander in Eingriff kommen, um ein Drehen des Rings (17) zu verhindern, die jedoch außer Eingriff bringbar sind, um ein Drehen des Rings (17) zu ermöglichen, wenn der Ring (17) gegen die Wirkung der Feder (19) angehoben wird.

16. Gerät gemäß Anspruch 15, wobei der Ring (17) ein doppelwandiges Strukturbauteil ist, das an einem Ende geschlossen ist, wodurch Luft zu den zweiten Einlassmitteln (16, 16') durch ein offenes Ende des Rings (17) zwischen den Wänden des Rings (17) zuführbar ist, und wobei die Innenwand des Rings (17) eine Öffnung aufweist, die zu den zweiten Einlassmitteln (16, 16') des Gehäuses (11) in oder außer Ausrichtung gebracht werden kann, wenn der Ring (17) gedreht wird.

17. Gerät gemäß Anspruch 15 oder 16, wobei die zweiten Einlassmittel eine Mehrzahl von Öffnungen (16, 16') umfassen, die um das Gehäuse (11) auf gleichem horizontalen Niveau unterhalb der Oberseite des Gehäuses (11) umfangsmäßig beabstandet sind, und wobei die Innenwand des Rings (17) eine Mehrzahl von Öffnungen aufweist, deren umfangsmäßige Beabstandung der der Mehrzahl der Öffnungen (16, 16') entspricht.

## Revendications

1. Appareil respiratoire à usage médical comprenant un logement (11) fabriqué à partir d'un premier matériau présentant une première entrée (13) destinée à se raccorder à une source de gaz sous pression, des seconds moyens formant entrée destinés à entraîner l'air et une sortie (22) destinée à amener le mélange gazeux à un patient, l'intérieur du logement (11) étant au moins en partie revêtu par une chicane tubulaire (26),
**caractérisé en ce que** la chicane tubulaire (26) est fabriquée à partir d'un second matériau présentant une densité supérieure à celle du premier matériau du logement (11), et la chicane tubulaire (26) fournit un passage en forme d'entonnoir pour le mélange entre les entrées (13, 16, 16') et la sortie (22).

2. Appareil selon la revendication 1, dans lequel le logement (11) et la chicane (26) sont tous les deux formés à partir d'une matière plastique.

3. Appareil selon la revendication 1 ou la revendication 2, dans lequel la chicane (26) est formée à partir d'une matière plastique contenant une matière de remplissage particulaire présentant une densité supérieure à celle de la matière plastique.

4. Appareil selon la revendication 3, dans lequel la chicane (26) est moulée à partir d'un composé plastique dont une plus grande proportion est une matière de remplissage en minerai de fer.

5. Appareil selon la revendication 4, dans lequel ledit composé présente une base de polypropylène et le composé représente approximativement 60% de minerai de fer sur 40% de polypropylène.

6. Appareil selon l'une quelconque des revendications précédentes, dans lequel l'épaisseur du matériau de la chicane se situe dans la plage de 2 à 3 mm.

7. Appareil selon l'une quelconque des revendications précédentes, dans lequel le matériau de la chicane (26) présente une densité de 1,4 à 2,4 g/cm³, et le matériau du logement (11) présente une densité de 0,95 à 1,13 g/cm³.

8. Appareil selon l'une quelconque des revendications précédentes, dans lequel la chicane (26) est fabriquée à partir d'un matériau présentant une densité au moins 30% supérieure à celle du logement (11).

9. Appareil selon l'une quelconque des revendications précédentes, dans lequel la première entrée (13) est située dans la partie supérieure du logement (11), la sortie (22) s'étend dans le sens radial par rapport au logement (11) entre les parties supérieure et inférieure de celui-ci et la seconde entrée (16, 16') étant disposée dans le sens radial par rapport au logement (11) entre la partie supérieure de celui-ci et la sortie (22), la chicane (26) présentant un composant généralement cylindrique (25) pour revêtir l'intérieur du logement (11) au-dessous de la partie supérieure de celui-ci et un composant généralement en forme de trompette (27) coaxial au composant cylindrique (25) et relié à celui-ci au niveau de la périphérie plus large de la partie en forme de trompette (27), la partie de diamètre plus étroit du composant en forme de trompette (27) s'étendant à distance de la partie supérieure du logement (11) au-delà des composants cylindriques (25).

10. Appareil selon la revendication 9, dans lequel une partie inférieure du logement (11) est un réservoir d'eau (12) d'où un tube (6) s'étend vers le haut à travers la chicane (26) en direction d'une buse (15) alignée sur le gaz arrivant à travers la première entrée (13), pour ajouter de ce fait de l'eau atomisée au mélange allant vers la sortie (22).

11. Appareil selon la revendication 9 ou la revendication 10, dans lequel la partie de diamètre plus étroite du composant en forme de trompette (27) de la chicane (26) présente une ouverture radiale (28) qui est tournée vers l'intérieur du logement (11) au niveau d'une position sensiblement diamétralement opposée à la sortie (22).

12. Appareil selon l'une quelconque des revendications 9 à 11, dans lequel la jonction entre les composants cylindrique (25) et en forme de trompette (27) de la chicane (26) se situe entre les extrémités supérieure et inférieure du composant cylindrique (25) et ce dernier est enlevé lorsqu'elles sont alignées toutes les deux sur la seconde entrée (16, 16') et sur la sortie (22).

13. Appareil selon l'une quelconque des revendications 9 à 12, dans lequel la partie du composant cylindrique (25) qui s'étend au-dessous de la jonction avec le composant en forme de trompette (27) présente des fentes longitudinales (31) dans une relation espacée de manière circonférentielle.

14. Appareil selon l'une quelconque des revendications 9 à 13, dans lequel la partie de diamètre plus étroit du composant en forme de trompette (27) termine dans une ouverture coaxiale (28) dimensionnée étroitement pour entourer le tube d'eau (6).

15. Appareil selon l'une quelconque des revendications précédentes, dans lequel les seconds moyens formant entrée (16, 16') peuvent être réglés par un ressort (17) pouvant tourner autour de la partie supérieure du logement (11), l'anneau (17) étant à ressort pour amener une extrémité inférieure de celui-ci en contact avec un épaulement (21) du logement (11), ladite extrémité inférieure et ledit épaulement (21) présentant des formations (20) qui se mettront mutuellement en prise pour empêcher une rotation de l'anneau (17) mais qui peuvent se dégager pour permettre la rotation de l'anneau (17) si l'anneau (17) est soulevé dans le sens contraire de l'action du ressort (19).

16. Appareil selon la revendication 15, dans lequel l'anneau (17) est une structure à deux parois, fermé à une extrémité, l'air étant admis en direction des seconds moyens formant entrée (16, 16') depuis une extrémité ouverte de l'anneau (17) entre les parois de l'anneau (17), la paroi interne de l'anneau (17) présentant une ouverture qui peut être amenée en alignement et hors de l'alignement avec les seconds moyens formant entrée (16, 16') du logement (11) lorsque l'anneau (17) est amené en rotation.

17. Appareil selon la revendication 15 ou la revendication 16, dans lequel les seconds moyens formant entrée comprennent une pluralité d'ouvertures (16, 16') espacées dans le sens circonférentiel autour du logement (11) au même niveau horizontal au-dessous de la partie supérieure du logement (11), et la paroi interne de l'anneau (17) présente une pluralité d'ouvertures dont l'espacement circonférentiel correspond à l'espacement circonférentiel de ladite pluralité d'ouvertures (16, 16').
